# EUROPÄISCHE PATENTSCHRIFT

(11) **EP 1 357 967 B1**
(45) Veröffentlichungstag und Bekanntmachung des Hinweises auf die Patenterteilung: **01.08.2012**
(21) Anmeldenummer: 02710048.6
(22) Anmeldetag: 29.01.2002
(51) Int. Cl.: A61M 16/00

(54) **Vorrichtung zur Zufuhr eines Atemgases**
Device for supplying a respiratory gas
Dispositif d'alimentation en gaz respiratoire

(30) Priorität: 29.01.2001 DE 10103810
(43) Veröffentlichungstag der Anmeldung: 05.11.2003
(60) Teilanmeldung: 10185080.8
(73) Patentinhaber: MAP Medizin-Technologie GmbH, 82152 Martinsried (DE)
(72) Erfinder: SCHNEIDER, Hartmut, Lutherville, MD 21093 (US); MEIER, Jörg, 81547 München (DE); HEIDMANN, Dieter, 82335 Berg (DE); VÖGELE, Harald, 82131 Gauting (DE); MADAUS, Stefan, 82152 Krailling (DE); JAKOBS, Rainer, 81539 München (DE); SCHÄTZL, Stefan, 82362 Weilheim (DE); BRANDMEIER, Richard, 82152 Martinsried (DE)
(74) Vertreter: Heunemann, Dieter
(86) Internationale Anmeldenummer: PCT/EP2002/000907
(87) Internationale Veröffentlichungsnummer: WO 2002/060518

(56) Entgegenhaltungen:
- WO-A-92/11054
- DE-A- 10 021 784

## Beschreibung

Die Erfindung betrifft eine Vorrichtung zur Zufuhr eines Atemgases zu einem Patienten im Rahmen der Diagnose und/oder der Therapie schlafbezogener Atmungsstörungen. Insbesondere betrifft die Erfindung ein CPAP-Gerät mit selbstabstimmender Bilevel-Druckregelung.

WO 92/11054 beschreibt ein solches CPAP-Gerät. Bearbeitung der Messsignale ergibt eine Ausgleichskurve aus pre-programmierten Steuerungs profilen. Das Therapie signal wird unter Berücksichtigung dieses Standardprofils generiert.

Schlafbezogene Atmungsstörungen, insbesondere in Verbindung mit Obstruktionen im Bereich der oberen Atemwege können auf physiologisch gut verträgliche Weise durch eine Überdruckbeatmung mit einem ggf. alternierenden, jedoch dauerhaft über dem Umgebungsdruckniveau liegenden Atemgasdruck therapiert werden. Diese allgemein als CPAP-Therapie bezeichnete Überdruckbeatmung basiert auf einer durch den Überdruck erreichten "pneumatischen Schienung" der oberen Atemwege. Durch diese pneumatische Schienung wird etwaigen Obstruktionen im Bereich der oberen Atemwege vorgebeugt. Um eine möglichst hohe physiologische Verträglichkeit der CPAP-Therapie zu gewährleisten, ist man bestrebt, den Beatmungsdruck möglichst gering zu halten und insbesondere während einer Expirationsphase gegenüber der Inspirationsphase abzusenken.

Günstige Beatmungsdruckpegel können bislang entweder durch den Patienten selbst gewählt werden oder beispielsweise auch unter ärztlicher Betreuung im Rahmen des Aufenthalts des Patienten in einem Schlaflabor ermittelt werden. Untersuchungen haben jedoch gezeigt, daß sowohl die vom Patienten selbst gewünschten, als auch die im Rahmen des Aufenthalts in einem Schlaflabor ermittelten Therapiedrücke für den Inspirationsdruck sowie für den üblicherweise etwas geringeren Expirationsdruck teilweise deutlich über den für den überwiegenden Teil der Schlafphase erforderlichen Inspirations- und Expirationsdruckpegeln liegen.

Unter dem Eindruck dieses Problems liegt der Erfindung liegt die Aufgabe zugrunde, eine Vorrichtung zur Zufuhr eines Atemgases zu einem Patienten zu schaffen, durch welche die Beatmungsdruckpegel noch zuverlässiger auf den physiologischen Zustand des Patienten abgestimmt werden können.

Diese Aufgabe wird wie beansprucht erfindungsgemäß gelöst durch eine Vorrichtung zur Zufuhr eines Atemgases mit einer elektronischen Signalverarbeitungseinrichtung zur Generierung eines Drucksteuerungssignales auf Grundlage von hinsichtlich der Atmungstätigkeit und/oder des physiologischen Zustandes einer Person indikativen Signalen , wobei die Signalverarbeitungseinrichtung eine Signaleingangseinrichtung und eine Extraktionseinrichtung aufweist, zur Generierung von Datenfeldeinträgen nach Maßgabe vorgegebener Signalanalyseprozeduren, und ein Drucksignalgenerator vorgesehen ist, zur Generierung des Drucksteuerungssignales , wobei der Drucksignalgenerator das Drucksignal unter Berücksichtigung wenigstens ausgewählter seitens der Extraktionseinrichtung determinierter Datenfeldeinträge generiert.

Dadurch wird es auf vorteilhafte Weise möglich, eine auf die momentanen physiologischen Bedürfnisse des Patienten verbessert abgestimmte Atemgasversorgung zu erreichen.

Gem. einer besonders bevorzugten Ausführungsform der Erfindung ist die Extraktionseinrichtung derart ausgebildet, daß durch diese eine Auswertung von hinsichtlich des Atemgasstromes indikativen Signalen erfolgt. Diese hinsichtlich des Atemgasstromes indikativen Signale können beispielsweise durch eine Staudrukkerfassungseinrichtung oder auch durch Meßschaltungen auf Grundlage akkustischer Prinzipien, insbesondere Dopplereffekt eines Ultraschallereignisses, generiert werden.

Die Auswertung der hinsichtlich des Atemgasstromes indikativen Signale erfolgt vorzugsweise auf Grundlage einer Zeitreihenanalyse, insbesondere auf Grundlage einer Varianzbetrachtung der ersten, zweiten und dritten Ableitung des Atemgasstromes.

Vorzugsweise ist die Extraktionseinrichtung derart ausgebildet, daß diese im Zusammenhang mit der Auswertung des Atemgasstromes die Abweichungen des Atemgasstromes von einem Referenzatemstrommuster analysiert. Dieses Referenzatemstrommuster kann kontinuierlich aktualisiert werden.

Es ist möglich, die Extraktionseinrichtung derart auszubilden, daß im Zusammenhang mit der Auswertung der hinsichtlich des Atemgasstromes indikativen Signale ein hinsichtlich des physiologischen Zustandes des Patienten indikativer Klassifikationsdatensatz generiert wird. Dieser Klassifikationsdatensatz kann über eine Schnittstelleneinrichtung, beispielsweise über eine Infrarotschnittstelle oder auch durch einen tragbaren Datenträger, einer weiteren Auswertung z.B. durch einen Facharzt, zugeführt werden.

Gem. einer besonders bevorzugten Ausführungsform der Erfindung wird seitens der Extraktionseinrichtung ein Verknüpfungsdatensatz generiert, der einen Zusammenhang zwischen den auf Grundlage ausgewählter Analysekriterien ermittelten Datenfeldeinträgen beschreibt. Dieser Zusammenhang zwischen den extrahierten Merkmalen oder die Wahrscheinlichkeit dieses Zusammenhangs kann durch statistische Angaben weiter klassifiziert werden, so daß die Bedeutung der extrahierten Merkmale, bzw. deren Gewichtung bei der Druckregelung entsprechend berücksichtigt werden kann.

Es ist möglich, auf Grundlage dieser statistischen Klassifikation der extrahierten Merkmale einen patientenspezifischen Datensatz zu generieren, aus welchem beispielsweise hervorgeht, daß bestimmte, in den Eingangssignalen enthaltene Informationen vernachlässigt werden können. So ist es beispielsweise möglich, anhand des patientenspezifischen Datensatzes zu beurteilen, in welcher Ausprägung ein Zusammenhang zwischen den Beatmungsdruckpegeln und der Körperposition des Patienten, insbesondere dem Halsverdrehungsgrad oder der Kopfposition, besteht. Es ist möglich, mit hoher statistischer Wahrscheinlichkeit auftretende Zusammenhänge statistisch zu beschreiben oder durch Parameter zu definieren.

Gem. einer besonders bevorzugten Ausführungsform der Erfindung ist der Drucksignalgenerator 4 derart operativ in die Signalverarbeitungseinrichtung derart eingebunden, daß dieser bei Generierung des Drucksteuerungssignales P Atemphasendaten sowie Atemphasenumschaltsignale berücksichtigt. Hierdurch wird es möglich, den zeitlichen Verlauf der Atemgasdruckänderung zwischen den abfolgenden Atemphasen derart abzustimmen, daß insbesondere während einer Einschlafphase und hohem Blutsauerstoffgehalt ein besonders hoher Beatmungskomfort gewährleistet ist. Die Atemphasenumschaltsignale werden gem. einer besonders bevorzugten Ausführungsform der Erfindung durch einen Atemphasenkoordinator erzeugt, der derart operativ in die Signalverarbeitungseinrichtung derart eingebunden ist, daß dieser in gleicher Weise wie der Drucksignalgenerator Zugriff auf bestimmte Feldeinträge des durch die Extraktionseinrichtung generierten Datenfeldes hat.

Der Atemphasenkoordinator ist vorzugsweise derart in die Signalvetarbeitungseinrichtung eingebunden, daß dieser auch im wesentlichen in Echtzeit Zugriff auf die an der Signaleingangseinrichtung anliegenden Signale "Rohdaten" oder zumindest davon abgeleiteter (beispielsweise definiert gefilterter) Signale hat. Dadurch wird es möglich, die Atemphasenerkennung unter einer präzisen Abstimmung auf den momentanen physiologischen Zustand des Anwenders vorzunehmen, ohne daß hierbei größere Verzugszeiten bei der Analyse des Atemgasstromes auftreten.

Gem. einer besonders bevorzugten Ausführungsform der Erfindung erfolgt die Atemphasenkoordination durch eine Atemphasenerkennung auf Grundlage einer Schwellwertbetrachtung, wobei der Schwellwert sich mit zunehmendem zeitlichen Abstand vom Zeitpunkt des zurückliegenden Atemphasenwechsels verändert. Diese Änderungsdynamik wird vorzugsweise auf Grundlage ausgewählter Einträge in dem Datenfeld programmgesteuert, abgestimmt. Die Atemphasenerkennung erfolgt vorzugsweise ebenfalls unter Rückgriffnahme auf einen umfangreicheren Datensatz, insbesondere ein Kennfeld, wobei die Position innerhalb dieses Kennfeldes in Abhängigkeit von dem momentan erkannten physiologischen Zustand des Anwenders ermittelt wird.

Der Atemphasenkoordinator ist vorzugsweise derart ausgebildet, daß dieser bei der Atemphasenerkennung die erste und vorzugsweise auch die zweite Ableitung des Atemgasstromes analysiert. Eine besonders hohe Zuverlässigkeit bzgl. der Erkennung der einzelnen Atemphasen kann dadurch erreicht werden, daß mehrere Erkennungskriterien verknüpft abgearbeitet werden. Es ist möglich, diese Erkennungskriterien auf Grundlage von FUZZI-Logik Prozeduren auszuwählen oder in Abhängigkeit von den durch die Extraktionseinrichtung generierten Datenfeldeinträgen unterschiedlich zu gewichten.

Der Atemphasenkoordinator und die Druckvorgabeeinrichtung sind vorzugsweise derart operativ verknüpft, daß im Falle unscharfer Atemphasenerkennung der Abstand zwischen dem Inspirationsdruck und dem Expirationsdruck verringert wird. Es ist möglich, die Druckvorgabeeinrichtung auch unter Rückgriffnahme auf einen zusätzlichen patientenspezifischen Druckregelungsdatensatz zu betreiben. Der Atemphasenselektionsdatensatz und der Druckregelungsdatensatz können aus Datenfeldern generiert werden, die unter Rückgriffnahme auf die seitens der Extraktionseinrichtung generierten Datenfeldeinträge modifiziert werden.

Gem. einer besonders bevorzugten Ausführungsform der Erfindung ist zur abschließenden Einstellung des Atemgasdruckes eine Druckabstimmungseinrichtung vorgesehen, welcher das seitens der Druckvorgabeeinrichtung generierte Drucksteuersignal als Sollgröße zugeführt wird. Die Druckabstimmungseinrichtung übernimmt nach Maßgabe des zugeführten Drucksteuersignales selbsttätig und vorzugsweise mit hoher Regeldynamik die Abstimmung des Atemgasdruckes.

Eine besonders präzise Einstellung des Atemgasdruckes wird hierbei dadurch erreicht, daß der Atemgasdruck an einer patientennahen Druckabgriffstelle erfaßt wird. Es ist möglich, den Atemgasdruck derart einzustellen, daß lediglich der statische Druckanteil an der Druckabgriffstelle, oder auch der statische Druckanteil im Innenraum einer Atemmaske dem nach Maßgabe des Drucksteuersignales vorgegebenen Atemgasdruck entspricht. Soweit ein unmittelbarer Druckabgriff im Bereich eines Anwenders nicht möglich ist, ist gem. einer besonders bevorzugten Ausführungsform der Erfindung die Druckabstimmungseinrichtung derart ausgebildet, daß diese unter Berücksichtigung des momentanen Atemgasstromes Druckkorrekturwerte zur Kompensation leitungsbedingter Druckabfälle errechnet.

Die Erfindungsgemäße Vorrichtung zur Zufuhr eines Atemgases eignet sich in besonderem Maße als CPAP-Gerät mit selbsttätig justierender Bilevel-Druckregelung.

Weitere Einzelheiten und Merkmale der Erfindung ergeben sich aus der nachfolgenden Beschreibung eines bevorzugten Ausführungsbeispieles in Verbindung mit der Zeichnung. Es zeigen:
- Fig. 1: eine Prinzipskizze zur Erläuterung des der Erfindung zugrundeliegenden regelungstechnischen Konzeptes;
- Fig. 2: ein mehrdimensionales, patientenspezifisch abgestimmtes Kennfeld, das den Zusammenhang zwischen dem Atemgasdruck und dem hiermit einhergehenden maximalen Atemgasstrom für unterschiedliche physiologische Zustände des Patienten darstellt;
- Fig. 3: ein Diagramm zur Erläuterung der in Abhängigkeit von einem Kriterienraster fortlaufend abgestimmten Beurteilungskriterien zur Erfassung der Atemzyklen eines Patienten sowie zur Abstimmung der Druckumschaltung.

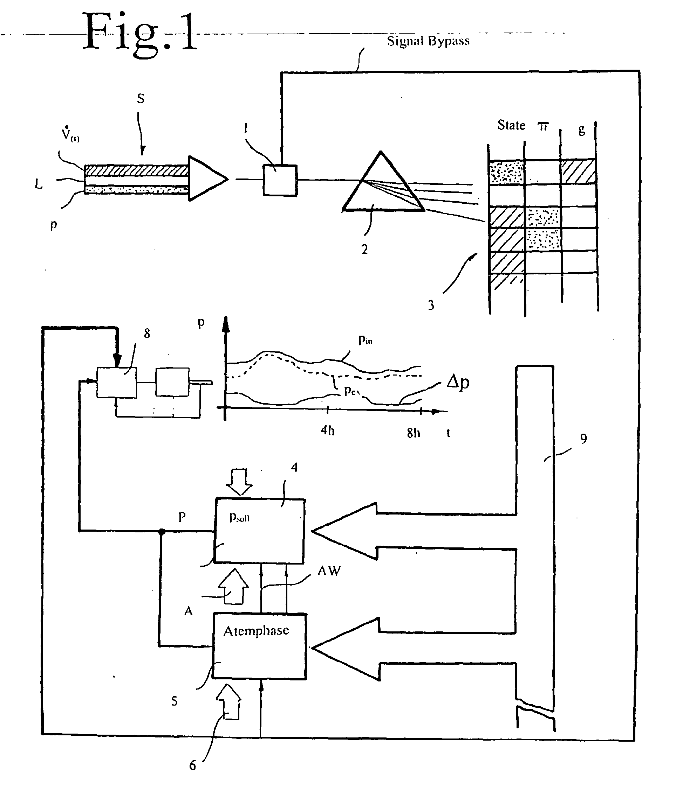

Gem. dem in Fig. 1 veranschaulichten regelungstechnischen Konzept werden hinsichtlich des momentanen physiologischen Zustands eines Patienten indikative Signale einer nachfolgend als Extraktionseinrichtung 2 bezeichneten Verarbeitungseinrichtung zugeführt. In dieser Extraktionseinrichtung 2 wird auf Grundlage einer adaptiven Auswerteprozedur ein Datenfeld 3 ausgefüllt. Die in dem Datenfeld 3 enthaltenen Informationen werden bei der Abstimmung des momentanen Zeitverhaltens der Atemgasdruckregelung berücksichtigt.

Bei dem hier gezeigten System wird ein Teil der Feldeinträge bei der Ermittlung eines Soll-Inspirationsdruckes sowie eines Soll-Expirationsdruckes berücksichtigt.

Diese Solldrücke werden in einem Atemphasenkoordinator 5 in Verbindung mit weiteren aus dem Kriterienfeld 3 ausgelesenen Einträgen verarbeitet. Der derart festgelegte momentane Atemgasdruckwert wird einer Drucksteuereinrichtung 8 zugeführt, die in Verbindung mit einem hochdynamischen Regelkreis den Atemgasdruck, beispielsweise durch Steuerung einer Gebläsedrehzahl auf den vorgegebenen Atemgassolldruck einstellt. Die extrem zeitnahe Einstellung der Atemgasdrücke wird bei dem gezeigten Regelungskonzept erreicht, indem zumindest ein Teil der patientenseitig abgegriffenen Signale S lediglich durch einen Bandpaß gefiltert, den einzelnen Regelungskomponenten zur Verfügung steht.

Das erfindungsgemäße Regelungskonzept kann durch eine ausreichend leistungsfähige Rechnereinrichtung realisiert werden, wobei es möglich ist, die hier klar abgegrenzt beschriebenen Komponenten in der Gestalt sich ggf. überlagernder Unterprozeduren durch einen entsprechenden Programmablauf zu verwirklichen.

In der Extraktionseinrichtung 2 wird vorzugsweise wenigstens ein hinsichtlich des momentanen Atemgasstromes indikatives Signal einer Zeitreihenanalyse unterzogen, wobei vorzugsweise wenigstens die erste, die zweite und die dritte Ableitung dieses Signals gebildet werden. Weiterhin werden statistische Parameter, insbesondere die Varianz des Signalverlaufes, bzw. dessen zeitliche Ableitungen ermittelt. Die im Zusammenhang mit den einzelnen Kurvendiskussionskriterien ermittelten Merkmale werden in dem Feld 3 vorzugsweise in Verbindung mit einer statistischen Sicherheit sowie einem sich ggf. mit zunehmender statistischer Aussagefähigkeit ändernden Gewichtungsfaktor oder Verknüpfungsparametern, eingetragen. Zumindest ein Teil der in das Feld 3 eingetragenen Angaben wird bei der Abstimmung des Zeitverhaltens der eigentlichen Atemgasdruckregelung berücksichtigt.

Bei dem hier gezeigten Ausführungsbeispiel werden über die Signaleingangseinrichtung 1 neben bzgl. des Atemflusses indikativen Signalen auch Lagesignale sowie hinsichtlich des Atemgasdruckes indikative Signale erfaßt. Diese Signale stehen in definiert gefilterter Form bedarfsweise den regelungstechnischen Systemen der erfindungsgemäßen Signalverarbeitungseinrichtung zur Verfügung. Durch die Extraktionseinrichtung 2 wird bei der hier dargestellten Ausführungsform ein mehrdimensionales Datenfeld 3 erzeugt. In diesem Datenfeld 3 befinden sich die sich dynamisch ändernden, durch die Extraktionseinrichtung generierten Datenfeldeinträge. Bei der gezeigten Ausführungsform werden bestimmten, durch die Extraktionseinrichtung 2 generierten Datenfeldeinträgen weitere Angaben zugeordnet, insbesondere Angaben zur statistischen Sicherheit. Die in dem nach Maßgabe der Extraktionseinrichtung 2 dynamisch verwalteten Datenfeld 3 abgelegten Datenfeldeinträge stehen, wie hier durch Datenflußpfeile 9 angedeutet, der Druckvorgabeeinrichtung 4 sowie dem Atemphasenkoordinator 5 zur Verfügung.

Die Druckvorgabeeinrichtung 4 beispielsweise ermittelt auf Grundlage, ausgewählter Datenfeldeinträge in dem dynamischen Datenfeld 3 den maximalen Inspirationsdruck sowie den maximalen Expirationsdruck. Weiterhin ermittelt die Druckvorgabeeinrichtung 4 einen, auf den momentanen physiologischen Zustand der spontan atmenden Person abgestimmten zeitlichen Druckverlauf beim Wechsel zwischen den beiden Atemphasen.

Die Erkennung der Atemphasen erfolgt über den Atemphasenkoordinator 5, der ebenfalls Zugriff auf ausgewählte Datenfeldeinträge in dem Datenfeld 3 hat. Der Atemphasenkoordinator 5 steht weiterhin mit der Signaleingangseinrichtung 1 in einem nahezu totzeitfreien Signalverbund. Die Beurteilung der Atmungstätigkeit der spontan atmenden Person durch den Atemphasenkoordinator 5 erfolgt bei dem dargestellten Ausführungsbeispiel zusätzlich unter Berücksichtigung patientenspezifischer Vorgabegrößen sowie auch unter Berücksichtigung der seitens der Druckvorgabeeinrichtung 4 vorgegebenen Drucksteuerungssignale P.

Hierdurch wird es möglich, bei Atemgaszufuhrperioden mit vergleichsweise großen Unterschieden zwischen dem Inspirationsatemgasdruck und dem Expirationsatemgasdruck die Atemphasenerkennung mit höherer Sensibilität vorzunehmen, wogegen bei vergleichsweise kleinen Differenzen zwischen dem Atemgasinspirationsdruck und dem Atemgasexpirationsdruck andere Kriterien der Erkennung bzw. der Bestimmung der Atemphasen zugrunde gelegt werden können.

Die seitens der Druckvorgabeeinrichtung 4 generierten Drucksteuerungssignale P werden einer Druckabstimmungseinrichtung 8 zugeführt, durch welche die für den Aufbau des Atemgasdruckes relevanten Organe, beispielsweise eine Gebläseeinrichtung, angesteuert werden. Die Regelung des Atemgasdruckes nach Maßgabe des Drucksteuerungssignales P kann über einen hierzu vorgesehenen hochdynamischen Regelkreis mit eigenen Druckerfassungsorganen erfolgen.

Im Zusammenspiel mit der beschriebenen, erfindungsgemäßen Signalverarbeitungseinrichtung wird es möglich, eine stabile und präzise auf die physiologischen Bedürfnisse einer spontan atmenden Person abgestimmte Einstellung des Atemgasdruckes zu gewährleisten.

Wie aus dem, in die Darstellung nach Fig. 1 eingefügten Diagramm hervorgeht, variieren der mittlere Inspirationsdruck und der mittlere Expirationsdruck einer Atemphase im Laufe einer sich über die ganze Schlafphase eines Patienten erstreckenden Zeitperiode. In Abhängigkeit von bestimmten, hinsichtlich des physiologischen Zustands der spontan atmenden Person indikativen Größen ergeben sich hierbei unterschiedlich große Druckabstände (Δp) zwischen dem Inspirationsdruck und dem mittleren Expirationsdruck jedes Atemzuges. In Abhängigkeit von dieser Druckdifferenz wird auch die Erkennung der Atemphasen durch den Atemphasenselektor 5 abgestimmt.

Die Abstimmung sowohl des Inspirationsdruckes als auch des Expirationsdruckes kann auf Grundlage eines patientenspezifisch adaptierten Kennfeldes erfolgen, wobei die momentane Position in diesem Kennfeld auf Grundlage von Klassifikationsangaben erfolgen kann, die im Zusammenspiel mit den durch die Extraktionseinrichtung 2 generierten Datenfeldeinträgen 3 ermittelt werden.

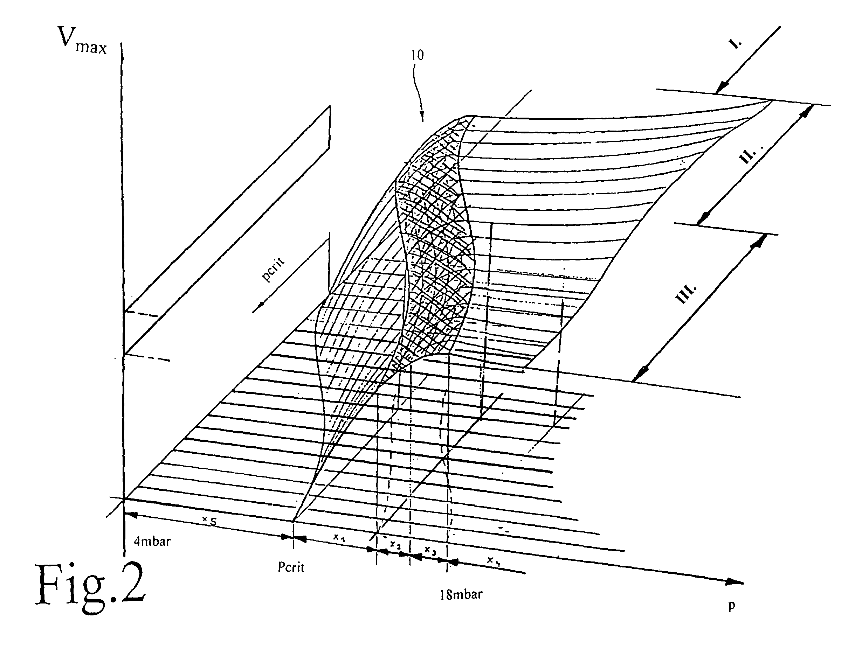

Bei dem in Fig. 2 dargestellten Ausführungsbeispiel wird anhand der in dem dynamischen Datenfeld 3 vorhandenen Datenfeldeinträge ermittelt, welcher Bereich des hier gezeigten Kennfeldes für die momentane Regelung des Atemgasdruckes heranzuziehen ist. Auf Grundlage einer derart getroffenen Auswahl des patientenspezifisch angepaßten Kennfeldbereiches wird ein maximaler Atemgasinspirationsdruck sowie hier zugehörig ein minimaler Atemgasexpirationsdruck ermittelt. Diese beiden Druckpegel liegen vorzugsweise innerhalb des hier stärker schraffiert angedeuteten Kniebereiches 10 des Kennfeldes. Im vorliegenden Beispiel erstreckt sich das Kennfeld über einen CPAP-Druckbereich von 4-18 mb. Der Abstand zwischen dem maximalen Inspirationsdruck dem minimalen Expirationsdruck liegt bei dem hier gezeigten patientenspezifischen Kennfeld unterhalb von 6 mb. Der Abstand zwischen dem maximalen Inspirationsdruck und dem minimalen Expirationsdruck wird neben der Steigung des Kennfeldes in dem dargestellten Kniebereich 10 auch durch weitere Einflußfaktoren bestimmt, die auf Grundlage des durch die Extraktionseinrichtung 2 dynamisch aufgefüllten Datenfeldes ermittelt werden.

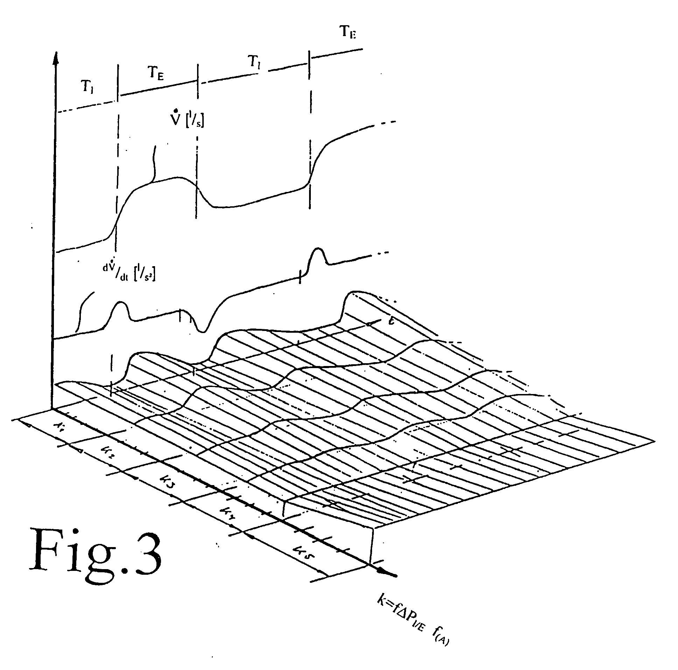

Die Darstellung gem. Fig. 3 zeigt qualitativ den zeitlichen Verlauf des Atemgasstromes V unter Zuordnung des seitens des Atemphasenkoordinators 5 erkannten Inspirationsphasen T_{I} und Expirationsphasen T_{E}. Unterhalb der Atemgasstromkurve ist die erste Ableitung derselben skizziert. In Abhängigkeit von den Einträgen in dem Datenfeld 3 sowie vorzugsweise auch in Abhängigkeit von den seitens der Druckvorgabeeinrichtung 4 vorgegebenen Drucksteuerungssignal P werden die Kriterien für die Atemphasenerkennung abgestimmt. Bei dem hier gezeigten Ausführungsbeispiel erfolgt in einer ersten Atemphasenklassifikationskategorie K₁ die Atemphasenerkennung durch einen Schwellwertvergleich mit einem sich zeitlich mit hoher Dynamik von einem vergleichsweise großen Wert zu einem relativ niedrigen Wert ändernden Schwellwert. Unmittelbar nach der Erkennung eines Atemphasenwechsels wird der Schwellwert wieder erhöht. Die unmittelbare Zurückschaltung in die vorangegangene Atemphase wird durch eine dynamisch angepaßte Totzeit vermieden. In der Klassifikationskategorie K₂ ist die Abweichung zwischen dem maximalen Schwellwert und dem minimalen Schwellwert bereits geringer. Noch geringer ist der Unterschied zwischen den beiden Schwellwerten in der Klassifikationskategorie 3. In der Klassifikationskategorie K₄ erfolgt die Atemphasenerkennung auf Grundlage eines nahezu konstanten Schwellwertes. In der Klassifikationskategorie K₅ erfolgt ebenfalls eine Atemphasenerkennung auf Grundlage konstanter, jedoch in Abhängigkeit von den Datenfeldeinträgen zunehmenden Schwellwerten.

Die Klassifikationskategorien werden vorzugsweise in Abhängigkeit von einer durch die Extraktionseinrichtung 2 durchgeführten und durch Datenfeldeinträge kommunizierten, Atemmustererkennung durchgeführt.

Durch die erfindungsgemäße Vorrichtung wird es möglich, ein CPAP-Gerät zur Durchführung einer Atemtherapie im häuslichen Bereich zu schaffen, das den Atemgasdruck sowohl für den Inspirationsvorgang als auch für den Expirationsvorgang selbsttätig auf die physiologischen Bedürfnisse eines Anwenders abstimmt.

Durch die erfindungsgemäß vorgesehene Extraktionseinrichtung wird es möglich, den momentanen physiologischen Zustand des Anwenders durch Erfassung der Atmungstätigkeit zu klassifizieren. Auf Grundlage dieser Klassifikation wird es möglich, die momentan günstigsten Atemgasdruckwerte für den Inspirationsvorgang und für den Expirationsvorgang festzulegen. Weiterhin wird es möglich, die für die Einstellung der ggf. abweichenden Druckpegel erforderliche Atemphasenerkennung mit hoher Präzision durchzuführen.

## Patentansprüche

1. Atemgaszufuhrvorrichtung mit einer elektronischen Signalverarbeitungseinrichtung, die ein Drucksteuerungssignal (P) auf Grundlage von hinsichtlich der Atmungstätigkeit und/oder des physiologischen Zustandes einer Person indikativen Signalen (S) generiert, wobei die Signalverarbeitungseinrichtung eine Signaleingangseinrichtung (1) und eine Extraktionseinrichtung (2) aufweist, die Datenfeldeinträge (3) nach Maßgabe vorgegebener Signalanalyseprozeduren generiert und ein Drucksignalgenerator (4) vorgesehen ist, der das Drucksteuerungssignal (P) generiert wobei der Drucksignalgenerator (4) das Drucksteuerungs signal (P) unter Berücksichtigung wenigstens ausgewählter seitens der Extraktionseinrichtung (2) determinierter Datenfeldeinträge (3) generiert.

2. Vorrichtung nach Anspruch 1, **dadurch gekennzeichnet, daß** die Extraktionseinrichtung (2) derart ausgebildet ist, daß durch diese eine Auswertung von hinsichtlich des Atemgasstromes indikativen Signalen erfolgt.

3. Vorrichtung nach Anspruch 1 oder 2, **dadurch gekennzeichnet, daß** die Extraktionseinrichtung (2) derart ausgebildet ist, daß die Auswertung der hinsichtlich des Atemgasstromes indikativen Signale auf Grundlage einer Zeitreihenanalyse erfolgt.

4. Vorrichtung nach wenigstens einem der Ansprüche 1 bis 3, **dadurch gekennzeichnet, daß** die Extraktionseinrichtung (2) derart ausgebildet ist, daß im Zusammenhang mit der Auswertung des Atemgasstromes die Abweichungen des Atemgasstromes von einem adaptiv aktualisierten Referenzatemstrommuster analysiert werden.

5. Vorrichtung nach wenigstens einem der Ansprüche 1 bis 4, **dadurch gekennzeichnet, daß** die Extraktionseinrichtung (2) derart ausgebildet ist, daß im Zusammenhang mit der Auswertung der hinsichtlich des Atemgasstromes indikativen Signale (S) ein hinsichtlich des physiologischen Zustandes des Patienten indikativer Klassifikationsdatensatz generiert wird.

6. Vorrichtung nach wenigstens einem der Ansprüche 1 bis 5 **dadurch gekennzeichnet, daß** die Extraktionseinrichtung (2) ausgebildet ist, einen Verknüpfungsdatensatz zu generieren, der einen Zusammenhang zwischen extrahierten Merkmalen beschreibt.

7. Vorrichtung nach wenigstens einem der Ansprüche 1 bis 6, **dadurch gekennzeichnet, daß** diese ausgebildet ist, unter Zwischenschaltung der Extraktionseinrichtung (2) der Zusammenhang zwischen extrahierten Merkmalen oder die Wahrscheinlichkeit des Zusammenhanges statistisch zu klassifizierten.

8. Vorrichtung nach wenigstens einem der Ansprüche 1 bis 7, **dadurch gekennzeichnet**, daβß diese ausgebildet ist, unter Zwischenschaltung der Extraktionseinrichtung (2) anhand einer statistischen Klassifikation der extrahierten Merkmale einen patientenspezifischen Datensatz zu generieren.

9. Vorrichtung nach wenigstens einem der Ansprüche 1 bis 8, **dadurch gekennzeichnet, daß** der Drucksignalgenerator (4) derart operativ in die Signalverarbeitungseinrichtung eingebunden ist, daß dieser bei der Generierung des Drucksignales (P) Atemphasendaten (A) und Atemphasenumschaltsignale (AW) berücksichtigt.

10. Vorrichtung nach wenigstens einem der Ansprüche 1 bis 9, **dadurch gekennzeichnet, daß** ein Atemphasenkoordinator (5) vorgesehen ist, zur Generierung von Atemphasendaten (A).

11. Vorrichtung nach wenigstens einem der Ansprüche 1 bis 10, **dadurch gekennzeichnet, daß** der Atemphasenkoordinator (5) operativ in die Signalverarbeitungseinrichtung derart eingebunden ist, daß dieser auf die Datenfeldeinträge (3) Zugriff hat.

12. Vorrichtung nach wenigstens einem der Ansprüche 1 bis 11, **dadurch gekennzeichnet, daß** der Atemphasenkoordinator (5)) operativ in die Signalverarbeitungseinrichtung derart eingebunden ist, daß dieser auf wenigstens einen Teil der an der Signaleingangseinrichtung (1) anliegenden Signale - oder davon abgeleiteter Signale Zugriff hat.

13. Vorrichtung nach wenigstens einem der Ansprüche 1 bis 12, **dadurch gekennzeichnet, daß** der Atemphasenkoordinator (5)) operativ in die Signalverarbeitungseinrichtung derart eingebunden ist, daß dieser auf einen patientenspezifischen Atemphasenselektionsdatensatz (6) Zugriff hat.

14. Vorrichtung nach wenigstens einem der Ansprüche 1 bis 13, **dadurch gekennzeichnet, daß** der Atemphasenkoordniator (5) derart ausgebildet ist, daß dieser eine Atemphasenerkennung auf Grundlage sich zeitlich ändernder Kriterien vornimmt.

15. Vorrichtung nach wenigstens einem der Ansprüche 1 bis 13, **dadurch gekennzeichnet, daß** der Atemphasenkoordinator (5) ausgebildet ist, eine Atemphasenerkennung in Verbindung mit einer Schwellwertbetrachtung vor zunehmen, wobei der Schwellwert sich mit zunehmendem zeitlichen Abstand von dem Zeitpunkt des zurückliegenden Atemphasenwechsel verändert.

16. Vorrichtung nach wenigstens einem der Ansprüche 1 bis 15, **dadurch gekennzeichnet, daß** der Atemphasenkoordinator (5) derart ausgebildet ist, daß der Schwellwert derart verändert wird, daß die Umschaltsensibilität zunimmt.

17. Vorrichtung nach wenigstens einem der Ansprüche 1 bis 16, **dadurch gekennzeichnet, daß** der Atemphasenkoordinator (5) derart ausgebildet ist, daß zur Atemphasenerkennung die erste und/oder die zweite Ableitung des Atemgasstromes analysiert wird.

18. Vorrichtung nach wenigstens einem der Ansprüche 1 bis 17, **dadurch gekennzeichnet, daß** der Atemphasenkoordinator (5) derart ausgebildet ist, daß im Rahmen der Atemphasenerkennung mehrere Erkennungskriterien verknüpft betrachtet werden.

19. Vorrichtung nach wenigstens einem der Ansprüche 1 bis 18, **dadurch gekennzeichnet, daß** der Atemphasenkoordinator (5) und der Drucksignalgenerator (4) derart miteinander operativ verknüpft sind, daß im Falle unscharfer Atemphasenerkennung der Abstand zwischen dem Inspirationsdruck und dem Expirationsdruck verringert wird.

20. Vorrichtung nach wenigstens einem der Ansprüche 1 bis 19, **dadurch gekennzeichnet, daß** der Drucksignalgenerator (4) auf einen patientenspezifischen Druckregelungsdatensatz (7) Zugriff hat.

21. Vorrichtung nach wenigstens einem der Ansprüche 1 bis 20, **dadurch gekennzeichnet, daß** der Atemphasenselektionsdatensatz (6) und/oder der'Druckregelungsdatensatz (7) unter Rückgriffnahme auf die seitens der Extraktionseinrichtung (2) generierten Datenfeldeinträge (3) generiert werden.

22. Vorrichtung nach wenigstens einem der Ansprüche 1 bis 21, **dadurch gekennzeichnet, daß** eine Druckabstimmungseinrichtung (8) vorgesehen ist, zur Einstellung des Atemgasdruckes nach Maßgabe des Drucksteuersignales.

23. Vorrichtung nach wenigstens einem der Ansprüche 1 bis 21, **dadurch gekennzeichnet, daß** die Druckabstimmungseinrichtung (8) den Atemgasdruck an einer patientennahen Druckabgriffsstelle erfaßt.

24. Vorrichtung nach wenigstens einem der Ansprüche 1 bis 23, **dadurch gekennzeichnet, daß** die Druckabstimmungseinrichtung (8) den Atemgasdruck derart abstimmt, daß dessen statischer Druckanteil an der Druckabgriffsstelle oder im Innenraum einer Atemmaske dem nach Maßgabe des Drucksteuersignales (P) vorgegebenen Atemgasdruck entspricht.

25. Vorrichtung nach wenigstens einem der Ansprüche 1 bis 24, **dadurch gekennzeichnet, daß** die Druckabstimmungseinrichtung (8) eine Druckkorrektureinrichtung umfaßt, zur Kompensation leitungsbedingter Druckabfälle.

26. CPAP-Gerät mit einer sich selbst auf die physiologischen Bedürfnisse eines Patienten abstimmenden Bilevel-Druckregelung bei welchem hinsichtlich des Atemgasstromes indikative Signale durch eine Extraktionseinrichtung analysiert werden und hierauf basierende Einträge in einem Datenspeicher vorgenommen werden, wobei ein Drucksignalgenerator und eine mit diesem operativ verbundene Atemphasenerkennungseinrichtung vorgesehen ist, und das Regelverhalten des Drucksignalgenerators sowie der Atemphasenerkennungseinrichtung auf Grundlage der Einträge in dem Datenspeicher dynamisch veränderbar sind.

## Claims

1. A respiratory gas supplying device comprising an electronic signal processing means generating a pressure control signal (P) on the basis of signals (S) that are indicative of the respiratory activity and/or the physiological state of a person, wherein the signal processing means comprises a signal input means (1) and an extraction means (2) generating data field entries (3) in accordance with predetermined signal analysis procedures and wherein a pressure signal generator (4) generating the pressure control signal (P) is provided, wherein the pressure signal generator (4) generates the pressure control signal (P) by taking into account at least selected data field entries (3) determined by the extraction means (2).

2. The device according to claim 1, **characterized in that** the extraction means (2) is configured such that it evaluates signals that are indicative of the respiratory gas flow.

3. The device according to claim 1 or 2, **characterized in that** the extraction means (2) is configured such that the evaluation of the signals that are indicative of the respiratory gas flow is based on a time series analysis.

4. The device according to at least one of claims 1 to 3, **characterized in that** the extraction means (2) is configured such that in connection with the evaluation of the respiratory gas flow, the deviations of the respiratory gas flow from an adaptively updated reference respiratory flow pattern are analyzed.

5. The device according to at least one of claims 1 to 4, **characterized in that** the extraction means (2) is configured such that in connection with the evaluation of the signals (S) that are indicative of the respiratory gas flow, a classification data record which is indicative of the physiological state of the patient is generated.

6. The device according to at least one of claims 1 to 5, **characterized in that** the extraction means (2) is configured to generate a linking data record which describes a relation between extracted features.

7. The device according to at least one of claims 1 to 6, **characterized in that** said device is configured to statistically classify the relation between extracted features or the probability of the relation by interconnecting the extraction means (2).

8. The device according to at least one of claims 1 to 7, **characterized in that** said device is configured to generate a patient-specific data record on the basis of a statistical classification of the extracted features by interconnecting the extraction means (2).

9. The device according to at least one of claims 1 to 8, **characterized in that** the pressure signal generator (4) is operatively integrated in the signal processing means such that it takes into account respiratory phase data (A) and respiratory phase shift signals (AW) when generating the pressure signal (P).

10. The device according to at least one of claims 1 to 9, **characterized in that** a respiratory phase coordinator (5) is provided for generating respiratory phase data (A).

11. The device according to at least one of claims 1 to 10, **characterized in that** the respiratory phase coordinator (5) is operatively integrated in the signal processing means such that it has access to the data field entries (3).

12. The device according to at least one of claims 1 to 11, **characterized in that** the respiratory phase coordinator (5) is operatively integrated in the signal processing means such that it has access to at least part of the signals in the signal input means (1) or signals derived therefrom.

13. The device according to at least one of claims 1 to 12, **characterized in that** the respiratory phase coordinator (5) is operatively integrated in the signal processing means such that it has access to a patient-specific respiratory phase selection data record (6).

14. The device according to at least one of claims 1 to 13, **characterized in that** the respiratory phase coordinator (5) is configured such that it performs a respiratory phase recognition on the basis of temporally changing criteria.

15. The device according to at least one of claims 1 to 13, **characterized in that** the respiratory phase coordinator (5) is configured to perform a respiratory phase recognition in connection with a consideration of a threshold value, wherein the threshold value changes as the time that has passed since the past respiratory phase change increases.

16. The device according to at least one of claims 1 to 15, **characterized in that** the respiratory phase coordinator (5) is configured such that the threshold is changed in such a manner that the shifting sensitivity increases.

17. The device according to at least one of claims 1 to 16, **characterized in that** the respiratory phase coordinator (5) is configured such that the first and/or the second derivative of the respiratory gas flow is/are analyzed for recognizing the respiratory phase.

18. The device according to at least one of claims 1 to 17, **characterized in that** the respiratory phase coordinator (5) is configured such that a plurality of recognition criteria are considered in a linked manner within the scope of the respiratory phase recognition.

19. The device according to at least one of claims 1 to 18, **characterized in that** the respiratory phase coordinator (5) and the pressure signal generator (4) are operatively linked in such a manner that in case of an indistinct respiratory phase recognition, the distance between the inspiration pressure and the expiration pressure is reduced.

20. The device according to at least one of claims 1 to 19, **characterized in that** the pressure signal generator (4) has access to a patient-specific pressure control data record (7).

21. The device according to at least one of claims 1 to 20, **characterized in that** the respiratory phase selection data record (6) and/or the pressure control data record (7) is/are generated by reverting to the data field entries (3) generated by the extraction means (2).

22. The device according to at least one of claims 1 to 21, **characterized in that** a pressure adjusting means (8) is provided for adjusting the respiratory gas pressure in accordance with the pressure control signal.

23. The device according to at least one of claims 1 to 21, **characterized in that** the pressure adjusting means (8) detects the respiratory gas pressure at a pressure measuring point close to the patient.

24. The device according to at least one of claims 1 to 23, **characterized in that** the pressure adjusting means (8) adjusts the respiratory gas pressure such that its statical pressure portion at the pressure measuring point or inside a breathing mask corresponds to the respiratory gas pressure that is predetermined in accordance with the pressure control signal (P).

25. The device according to at least one of claims 1 to 24, **characterized in that** the pressure adjusting means (8) comprises a pressure correction means for compensating conduit-caused pressure reductions.

26. A CPAP device which comprises a bilevel pressure control adjusting automatically to the physiological requirements of a patient and in which signals that are indicative of the respiratory gas flow are analyzed by an extraction means and entries that are based thereon are made in a data storage, wherein a pressure signal generator is provided as well as a respiratory phase recognition means that is operatively connected thereto, and wherein the control behavior of the pressure signal generator as well as of the respiratory phase recognition means can be changed dynamically on the basis of the entries in the data storage.

## Revendications

1. Dispositif d'alimentation en gaz respiratoire comportant un dispositif électronique de traitement de signal; qui génère un signal de commande de pression (P) sur la base de signaux (S) indicatifs concernant l'activité respiratoire et/ou l'état physiologique d'une personne, dans lequel le dispositif de traitement de signal présente un dispositif d'entrée de signal (1) et un dispositif d'extraction (2) qui génèrent des entrées de champs de données (3) en application de procédures d'analyse de signal prédéterminées et dans lequel est prévu un générateur de signal de pression (4) qui génère le signal de commande de pression (P), le générateur de signal de pression (4) générant le signal de commande de pression (P) en tenant compte d'au moins des entrées de champs de données (3) choisies, déterminées au niveau du dispositif d'extraction (2).

2. Dispositif selon la revendication 1, **caractérisé en ce que** le dispositif d'extraction (2) est conçu de telle sorte que par le biais de celui-ci, une évaluation des signaux indicatifs concernant le débit de gaz respiratoire s'effectue.

3. Dispositif selon la revendication 1 ou 2, **caractérisé en ce que** le dispositif d'extraction (2) est conçu de telle sorte que l'évaluation des signaux indicatifs concernant le débit de gaz respiratoire s'effectue sur la base d'une analyse des séries temporelles.

4. Dispositif selon au moins l'une quelconque des revendications 1 à 3, **caractérisé en ce que** le dispositif d'extraction (2) est conçu de telle sorte que, dans le cadre de l'évaluation du débit de gaz respiratoire, les écarts du débit de gaz respiratoire par rapport à un modèle de débit respiratoire de référence actualisé adaptativement soient analysés.

5. Dispositif selon au moins l'une quelconque des revendications 1 à 4, **caractérisé en ce que** le dispositif d'extraction (2) est conçu de telle sorte que, dans le cadre de l'évaluation des signaux indicatifs (S) concernant le débit de gaz respiratoire, un jeu de données de classification indicatif concernant l'état physiologique du patient soit généré.

6. Dispositif selon au moins l'une quelconque des revendications 1 à 5, **caractérisé en ce que** le dispositif d'extraction (2) est conçu pour générer un jeu de données de connexion qui décrit une relation entre des caractéristiques extraites.

7. Dispositif selon au moins l'une quelconque des revendications 1 à 6, **caractérisé en ce qu'**il est conçu pour établir une classification statistique, par interposition du dispositif d'extraction (2), du rapport entre des caractéristiques extraites ou de la probabilité du rapport.

8. Dispositif selon au moins l'une quelconque des revendications 1 à 7, **caractérisé en ce qu'**il est conçu pour générer, par interposition du dispositif d'extraction (2), un jeu de données spécifiques du patient sur la base d'une classification statistique des caractéristiques extraites.

9. Dispositif selon au moins l'une quelconque des revendications 1 à 8, **caractérisé en ce que** le générateur de signal de pression (4) est intégré fonctionnellement dans le dispositif de traitement de signal, de telle sorte que celui-ci prenne en compte lors de la génération du signal de pression (P), les données de phases respiratoires (A) et les signaux de phase respiratoire (AW).

10. Dispositif selon au moins l'une quelconque des revendications 1 à 9, **caractérisé en ce qu'**un coordonnateur de phases respiratoires (5) est prévu pour la génération des données de phases respiratoires (A).

11. Dispositif selon au moins l'une quelconque des revendications 1 à 10, **caractérisé en ce que** le coordinateur de phases respiratoires (5) est intégré fonctionnellement dans le dispositif de traitement de signal de telle sorte que celui-ci ait un accès à l'entrée de champ de données (3).

12. Dispositif selon au moins l'une quelconque des revendications 1 à 11, **caractérisé en ce que** le coordinateur de phases respiratoires (5) est intégré fonctionnellement dans le dispositif de traitement de signal de telle sorte que celui-ci ait un accès à au moins une partie des signaux dans le dispositif d'entrée de signal (1) ou aux signaux dérivés de celui-ci.

13. Dispositif selon au moins l'une quelconque des revendications 1 à 12, **caractérisé en ce que** le coordinateur de phases respiratoires (5) est intégré fonctionnellement dans le dispositif de traitement de signal de telle sorte que celui-ci ait un accès à un jeu de données de sélection de phases respiratoires (6) spécifiques du patient.

14. Dispositif selon au moins l'une quelconque des revendications 1 à 13, **caractérisé en ce que** le coordinateur de phases respiratoires (5) est conçu de telle sorte que celui-ci effectue une reconnaissance des phases respiratoires sur la base de critères changeants temporellement.

15. Dispositif selon au moins l'une quelconque des revendications 1 à 13, **caractérisé en ce que** le coordinateur de phases respiratoires (5) est conçu pour effectuer une reconnaissance des phases respiratoires en relation avec la prise en compte d'une valeur seuil, la valeur seuil se modifiant lorsque l'intervalle temporel augmente à partir du moment du changement de phases respiratoires antérieur.

16. Dispositif selon au moins l'une quelconque des revendications 1 à 15, **caractérisé en ce que** le coordinateur de phases respiratoires (5) est conçu de telle sorte que la valeur seuil soit modifiée de telle sorte que la sensibilité d'inversion augmente.

17. Dispositif selon au moins l'une quelconque des revendications 1 à 16, **caractérisé en ce que** le coordinateur de phases respiratoires (5) est conçu de telle sorte que pour la reconnaissance des phases respiratoires, la première différentiation et/ou la deuxième différentiation du débit de gaz respiratoire soit analysée.

18. Dispositif selon au moins l'une quelconque des revendications 1 à 17, **caractérisé en ce que** le coordinateur de phases respiratoires (5) est conçu de telle sorte que dans le cadre de la reconnaissance des phases respiratoires, plusieurs critères de reconnaissances soient pris en compte d'un manière reliée.

19. Dispositif selon au moins l'une quelconque des revendications 1 à 18, **caractérisé en ce que** le coordinateur de phases respiratoires (5) et le générateur de signal de pression (4) sont reliés fonctionnellement l'un à l'autre de telle sorte que dans le cas de reconnaissance de phases respiratoires imprécise, l'intervalle entre la pression d'inspiration et la pression d'expiration soit réduit.

20. Dispositif selon au moins l'une quelconque des revendications 1 à 19, **caractérisé en ce que** le générateur de signal de pression (4) a accès à un jeu de données de régulation de pression (7) spécifiques du patient.

21. Dispositif selon au moins l'une quelconque des revendications 1 à 20, **caractérisé en ce que** le jeu de données de sélection des phases respiratoires (6) et/ou le jeu de données de régulation de pression (7) est généré en recourant aux entrées de champs de données (3) générées au niveau du dispositif d'extraction (2).

22. Dispositif selon au moins l'une quelconque des revendications 1 à 21, **caractérisé en ce qu'**un dispositif de détermination de pression (8) est prévu pour ajuster la pression de gaz respiratoire en fonction du signal de commande de pression.

23. Dispositif selon au moins l'une quelconque des revendications 1 à 21, **caractérisé en ce que** le dispositif de détermination de pression (8) saisit la pression de gaz respiratoire sur un site de prise de pression à proximité du patient.

24. Dispositif selon au moins l'une quelconque des revendications 1 à 23, **caractérisé en ce que** le dispositif de détermination de pression (8) détermine la pression de gaz respiratoire de telle sorte que sa proportion de pression statique sur le site de prise de pression ou à l'intérieur d'un masque respiratoire corresponde à la pression de gaz respiratoire prédéterminée selon le signal de commande de pression (P).

25. Dispositif selon au moins l'une quelconque des revendications 1 à 24, **caractérisé en ce que** le dispositif de détermination de pression (8) comprend un dispositif de correction de pression pour compenser les chutes de pression dues à la conduite.

26. Appareil de CPAP comportant un réglage de pression à deux niveaux se déterminant lui-même en fonction des besoins physiologiques d'un patient, dans lequel des signaux indicatifs concernant le débit de gaz respiratoire sont analysés par un dispositif d'extraction et des entrées basées sur ceux-ci sont effectuées dans une mémoire de données, un générateur de signal de pression et un dispositif de reconnaissance de phases respiratoires relié fonctionnellement à celui-ci étant prévus, et le comportement de régulation du générateur de signal de pression et du dispositif de reconnaissance de phases respiratoires étant modifiables de façon dynamique sur la base des entrées dans la mémoire de données.
